# EUROPÄISCHE PATENTSCHRIFT

(11) **EP 0 680 292 B1**
(45) Veröffentlichungstag und Bekanntmachung des Hinweises auf die Patenterteilung: **21.07.1999**
(21) Anmeldenummer: 95903272.3
(22) Anmeldetag: 23.11.1994
(51) Int. Cl.: A61F 2/38, A61F 2/46

(54) **SYSTEM FÜR DIE AUSBILDUNG EINER KNIEGELENK-ENDOPROTHESE**
SYSTEM FOR CONSTRUCTING A KNEE-JOINT ENDOPROSTHESIS
SYSTEME PERMETTANT DE REALISER UNE ENDOPROTHESE POUR L'ARTICULATION DU GENOU

(30) Priorität: 23.11.1993 DE 4339895; 19.07.1994 DE 4425529
(43) Veröffentlichungstag der Anmeldung: 08.11.1995
(73) Patentinhaber: Gerber, Bruno E., Dr., 2007 Neuchâtel (CH)
(72) Erfinder: Gerber, Bruno E., Dr., 2007 Neuchâtel (CH)
(74) Vertreter: Popp, Eugen, Dr.
(86) Internationale Anmeldenummer: EP9403875
(87) Internationale Veröffentlichungsnummer: WO9514444

(56) Entgegenhaltungen:
- EP-A- 0 327 297
- EP-A- 0 498 586
- EP-A- 0 519 872
- EP-A- 0 519 873
- GB-A- 2 184 025

## Beschreibung

Die Erfindung betrifft ein System für die Ausbildung einer Kniegelenk-Endoprothese mit
- am unteren Ende des Oberschenkels (Femur) befestigbaren femoralen Gelenkelementen mit konvex gekrümmter Gelenklagerfläche,
- am oberen Ende des Schienbeins (Tibia) befestigbaren, separat ausgebildeten, tibialen Lagerelementen, und mit
- je einem zwischen den femoralen Gelenk- und den tibialen Lagerelementen beweglich angeordneten Meniscus mit auf der Oberseite und der Unterseite ausgebildeten Gleitflächen, welche komplementär zu der zugeordneten Gelenklagerfläche des femoralen Gelenkelementes bzw. der zugeordneten Lagerfläche des tibialen Lagerelementes gestaltet sind.

Eine Kniegelenk-Endoprothese mit den vorgenannten Merkmalen ist bekannt aus der DE 25 50 704 C2. Die vorgeschlagene Kniegelenk-Endoprothese ist so aufgebaut, daß sie die Geometrie des "natürlichen" Kniegelenks und seiner Lagerflächen weitgehend imitiert. Zwischen dem Gelenk- und dem Lagerelement ist ein bewegliches Meniscus-Element vorgesehen, dessen auf der Ober- und Unterseite angeordnete Gleitflächen eine komplementäre Gestaltung einerseits zu der Gelenklagerfläche des femoralen Gelenkelementes und andererseits zur Lagerfläche des tibialen Lagerelementes aufweisen. Bei einem derartigen Aufbau wird das natürliche Spiel der Muskeln und Bänder relativ wenig gestört und gleichzeitig eine relativ gute Verteilung der Belastung sichergestellt. Das Gelenk- und das Meniscus-Element einerseits und das Meniscus- und das Lagerelement andererseits können sich jeweils relativ unabhängig voneinander bewegen, weil die zwei gewissermaßen mechanisch in Reihe geschalteten Teilgelenke jeweils komplementär gestaltete Lagerflächen aufweisen. Insbesondere können sich das Gelenk- und das Meniscus-Element relativ zueinander um drei orthogonale Achsen drehen, und das Meniscus-Element und das Lagerelement können in zwei dieser Richtungen relativ zueinander gleiten und um die dritte dieser Achsen zueinander drehen. Die sich daraus ergebende Bewegungsfähigkeit zwischen dem Gelenk- und dem Lagerelement umfaßt daher sowohl ein Rollen, ein Gleiten und ein Verdrehen um diejenigen Kombinationen dieser Bewegungsarten, die man beim natürlichen Knie findet. Die im wesentlichen konvexen und relativ flachen Gestaltungen der beiden Lagerflächen können so nahe an die Oberflächengestaltungen der natürlichen Gelenkteile der zugeordneten Einzelelemente des Knies angeglichen werden, daß auch das Zusammenwirken mit den das Gelenk umgebenden Muskeln und Bändern wie im natürlichen Knie abläuft. Eine geteilte Ausführung sowohl der femoralen Gelenkelemente als auch der tibialen Lagerelemente ermöglicht die Herstellung von modular ins Prothesen-System eingepaßten Halbprothesen. Insofern unterscheidet sich die Kniegelenk-Endoprothese gemäß der DE 25 50 704 C2 auch vorteilhaft von der Kniegelenk-Endoprothese gemäß der EP 0 519 873 A2 oder der EP 0 519 872 A1.

Nachteilig bei den bekannten Systemen ist, daß schräg nach außen in die Tibiaplateaus einstrahlende Belastungskräfte perpendikulär durch das Implantat nicht abgefangen werden können. Dementsprechend zeichnen sich die bekannten Konstruktionen dadurch aus, daß die seitliche Stabilität nur durch die Suffizienz der natürlichen Seitenbänder garantiert wird.

Der vorliegenden Erfindung liegt daher die Aufgabe zugrunde, ein System der eingangs genannten Art zu schaffen, bei dem schräg in die Tibia-Plateaus einstrahlende Belastungskräfte mit einfachsten Mitteln wirkungsvoll abgefangen werden können, so daß die inhärente Stabilität der Kniegelenk-Endoprothese diesbezüglich im Vergleich zum Stand der Technik erhöht wird. Gleichzeitig sollen die erwähnten Vorteile der Kniegelenk-Endoprothese gemäß der DE 25 50 704 C2 beibehalten werden.

Diese Aufgabe wird erfindungsgemäß durch die kennzeichnenden Merkmale des Anspruches 1 gelöst.

Dementsprechend liegt die sagittal äußere Begrenzung der den femoralen Gelenkelementen zugewandten Gleitflächen der Menisci höher als deren sagittal innere Begrenzung und sind die Menisci unterschiedlich hoch ausgebildet. Diese Anordnung der tibialen Lagerelemente stellt eine deutliche Abkehr von der bisherigen Praxis dar mit dem Effekt, daß das Kniegelenk flexibler an die tatsächlich auftretenden, individuell unterschiedlich ausgebildeten Kräfteverhältnisse angepaßt werden kann. Natürliche Kniegelenke sind individuell sehr unterschiedlich ausgebildet, insbesondere der in der vertikalen Querebene liegende Winkel zwischen Ober- und Unterschenkel, worauf beispielsweise die umgangssprachlichen Begriffe "X-Beine" und "O-Beine" hinweisen. Demzufolge sind auch die Richtungen der auf das Schienbein wirkenden Kräfte unterschiedlich ausgebildet.

Vorteilhafte konstruktive Weiterbildungen der Erfindung sind in den Unteransprüchen beschrieben. Eine nutartige Gleitführung an der Oberseite der tibialen Lagerelemente ermöglicht eine Querbewegung der Menisci relativ zu den tibialen Lagerelementen, wie es auch beim natürlichen Knie der Fall ist.

Vorteilhafterweise sind die Lagerflächen der tibialen Lagerelemente jeweils sphärisch-konkav gewölbt.

Auf diese Weise sind die Menisci allseits sphärisch definiert, wodurch nicht nur eine antero-posteriore Beweglichkeit, sondern auch seitliche Beweglichkeit der Menisci möglich wird. Damit ist neben der oben erwähnten erhöhten Stabilität der erfindungsgemäßen Kniegelenk-Endoprothese zugleich eine erhöhte Beweglichkeit derselben in Anpassung an ein natürliches Kniegelenk erreicht. Bei dieser doppel-sphärischen Ausbildung der Menisci stellen sich diese in jeder Beugestellung des Knies optimal relativ zum zugeordneten femoralen Gelenkelement ein.

Vorzugsweise ist auch die Unterseite der tibialen Lagerelemente sphärisch-konvex gewölbt. Die entsprechend ausgebildeten Tibia-Implantate werden dann mit einem sogenannten Impaktor bzw. Schlaggerät kortikal aufliegend in gewünschter Neigung eingeschlagen, wobei durch Spongiosa-Impaktion das Implantat-Bett von selbst die erforderliche Paßform erhält, und zwar ohne unnötige Knochenentfernung.

Nachteilig bei den bekannten Systemen ist auch noch, daß die Implantation der tibialen Lagerelemente eine besonders hohe Paßgenauigkeit erfordert, wenn eine Schaukelbelastung der Implantatverankerungsflächen vermieden werden soll, welche bei einem einzigen Tibiaflächenimplantat ohnehin nicht ganz eliminierbar ist. Sehr häufig ist jedoch eine Nachkorrektur erforderlich, die dann entweder unterlassen wird oder nur unter Inkaufnahme eines größeren tibialen Knochenstückverlustes möglich ist. Vor allem müssen die Lagerelemente unter Hinterlassung großer Zapfenlöcher wieder entfernt werden mit der Folge, daß der Knochen erheblich darunter leidet. Dabei ist zu bedenken, daß die Stabilität von Knochen älterer Menschen nicht mehr allzu groß ist. Ein Heraushebeln und erneutes Einsetzen der implantierten Lagerelemente hat eine erhebliche Schwächung des ohnehin angegriffenen Knochens zur Folge. Zur Lösung dieser Problematik sind entsprechend den Ansprüchen 8 ff tibiale Probe-Lagerelemente mit zugehörigen Probe-Menisci vorgeschlagen, wobei an der Unterseite der Probe-Lagerelemente mindestens ein kleiner Knochenhaftdorn vorgesehen ist. Dementsprechend wird nach dieser Vorgabe mit tibialen Probe-Lagerelementen und Probe-Menisci gearbeitet. Der bzw. die Knochenhaftdorne an der Unterseite der Probe-Lagerelemente sollen diese lediglich so lange gegen seitliches Abgleiten in Position halten, bis eine endgültige Lage des bzw. der Probe-Lagerelemente und damit entsprechenden endgültigen Lagerelemente festliegt. Mittels eines Zielgeräts wird versucht, das Probe-Lagerelement hinsichtlich seiner Längs- und Seitenneigung sowie Höhe optimal einzustellen, wobei zu diesem Zweck das Probe-Lagerelement unter Umständen mehrmals wieder entfernt werden muß, um die Auflagefläche am Knochen mittels einer Säge oder dergleichen Werkzeug nacharbeiten zu können so lange, bis die richtige Lage für das Probe-Lagerelement gefunden ist, in welcher es während des ganzen Verlaufs von Beugung und Streckung des Knies unverändert voll aufliegt. Das endgültig zu implantierende Lagerelement entspricht hinsichtlich der Abmessungen exakt dem Probe-Lagerelement. Wenn dementsprechend für das Probe-Lagerelement die richtige Position im Verhältnis zu dem zugeordneten femoralen Gelenkelement gefunden worden ist, braucht das Probe-Lagerelement lediglich durch das endgültig zu implantierende Lagerelement ersetzt zu werden. Das endgültig zu implantierende Lagerelement weist wesentlich tiefer in den Knochen eindringende Verankerungsmittel auf. Dies ist jedoch nicht weiter schädlich, da das endgültig zu implantierende Lagerelement nur einmal in der vorher mittels des entsprechenden Probe-Lagerelements gefundenen optimalen Position implantiert wird. Die an der Unterseite des Probe-Lagerelements angeordneten Knochenhaftdorne dringen nicht sehr tief in den Knochen ein. Dementsprechend gering ist die Einwirkung auf den Knochen, so daß mehrmaliges Entfernen und erneutes Einsetzen eines Probe-Lagerelements keine negativen Auswirkungen auf den Knochen haben. Durch die gemäß Anspruch 7 vorgeschlagene Wölbung der Unterseite der tibialen Lagerelemente erhalten diese eine zusätzliche Fixierung am proximalen Ende der Tibia. Es sei noch darauf hingewiesen, daß die Maßnahmen nach den Ansprüchen 8 ff auch als unabhängige Erfindung beansprucht werden.

Dadurch, daß die tibiale Lagerung der Menisci zweigeteilt ist, können die entsprechenden Lagerflächen unabhängig voneinander eingestellt werden. Dies gilt sowohl hinsichtlich ihrer Längs- und Seitenneigung als auch hinsichtlich ihres Abstandes vom linken und rechten femoralen Gelenkelement, wobei diese beiden Gelenkelemente auch zu einem einzigen Doppelkufen-Femurschlitten zusammengefaßt sein können. Aufgrund der fehlenden starren Verbindung zwischen den beiden tibialen Implanteten tritt auch nicht das unerwünschte "Schaukeln" der tibialen Lagerung auf, wie dies bei einstückiger Ausführung sehr häufig der Fall ist. Dieses "Schaukeln" führt auch zu einer Lockerung des tibialen Implantats mit der Folge, daß dieses oftmals nach relativ kurzer Zeit wieder ausgetauscht werden muß.

Das erfindungsgemäße System ist darüber hinaus gekennzeichnet durch Interkompatibilität zwischen femoralen Gelenkelementen, tibialen Lagerelementen und den Menisci aus vorzugsweise Polyethylen oder dergleichen Material. Das beschriebene System eignet sich für alle Versorgungsstufen, d. h. für eine femoral-tibiale oder femoral-patellare Versorgung, insbesondere eine sogenannte Teil- oder Halbprothese, Vollprothese und auch Revisionsprothese.

Von besonderer Bedeutung ist noch die Ausbildung einer nutartigen Gleitführung für die Probe- bzw. endgültig verwendeten Menisci an der Oberseite der Probe-Lagerelemente ebenso wie an der Oberseite der endgültig implantierten tibialen Lagerelemente. Vorzugsweise ist die nutartige Gleitführung um die Schienbein-Längsachse bzw. Knie-Querachse herum gebogen. Diese "wannen- bzw. schalenförmige" Aufnahme der Gleitführung für die Menisci hat zur Folge, daß die Menisci über die gesamte Breite relativ hoch gebaut werden können. Dementsprechend steht relativ viel Polyethylen-Material zur Abstützung der femoralen Gelenkelemente zur Verfügung. Im übrigen werden die Menisci auf der Lagerfläche der tibialen Lagerelemente durch die nutartige Gleitführung gehalten. Diese Gleitführung ist vorzugsweise entweder gemäß Anspruch 10 oder gemäß den Ansprüchen 11 und/oder 12 gestaltet.

Die Probe-Menisci können ebenso wie die endgültig implantierten Menisci unterschiedliche Höhen aufweisen. Dementsprechend können je nach Abnutzungsgrad unterschiedliche Abstände zwischen den tibialen Lagerelementen und den zugeordneten femoralen Gelenkelementen ausgeglichen werden.

Die den Probe-Lagerelementen entsprechenden endgültig implantierten Lagerelement weisen an ihrer Unterseite jeweils mindestens ein zapfen- oder laschenförmiges, vorzugsweise hülsenförmiges Verankerungselement mit mindestens einer, insbesondere zwei übereinander angeordneten Querbohrungen auf. Das vorgenannte Verankerungselement erstreckt sich zum Zwecke der besseren Verankerung der Lagerelements vorteilhafterweise schräg zur Mittenachse desselben, und zwar vorzugsweise unter einem Winkel von 5 bis 20°. Bei Ausbildung von zwei Verankerungselementen an der Unterseite des endgültig implantierten Lagerelements können sich diese vorzugsweise parallel zueinander erstrecken. Es ist auch von Vorteil, wenn die Hülsenwand der Verankerungselemente nach Art einer Schneide ausgebildet ist. Dem beschriebenen System ist also immanent, daß den erwähnten Probe-Menisci und Probe-Lagerelementen entsprechende Menisci und Lagerelemente zur endgültigen Implantation zur Verfügung gestellt sind.

Die Probe-Lagerelemente weisen im Bereich ihrer Meniscus-Gleitflächen jeweils noch eine Bohrung zur Führung eines Knochenbearbeitungswerkzeuges, insbesondere eines Zylinderhohlmeißels, auf. Durch diese Bohrung kann also die entsprechende Aufnahmebohrung für das an der Unterseite des endgültig implantierten Lagerelements angeordnete Verankerungselement ausgebildet werden. Auch diesbezüglich ist das Probe-Lagerelement für den Operateur besonders vorteilhaft. Die vorgenannte Führungsbohrung erstreckt sich vorzugsweise in einer Neigung, die der Neigung des Verankerungselements an der Unterseite des endgültig implantierten Lagerelements entspricht.

Die erwähnte wannenförmige Ausbildung des Lagerelements hat noch den Vorteil, daß dieses nicht nur mit seiner Unterseite, sondern auch mit seiner Innenseite am entsprechend bearbeiteten Knochen anliet. Die Position des tibialen Implantats wird auf diese Weise dauerhaft auch gegen Rotation gesichert. Da das eingangs erwähnte "Schaukeln" ausbleibt, wird eine Zugbelastung des Interface durch Abheben des tibialen Lagerelements vemieden. Somit kann eine deutliche Lockerungstendenz der tibialen Lagerelemente auch nach längerem Einsatz und starker Belastung erwartet werden.

Anstelle der femoralen Gelenkelemente kann bei Bedarf auch ein Patella-Gleitlager zum Einsatz kommen.

Das beschriebene System stellt sich grundsätzlich als echt sphäroide unikompartimentale Schlitten-Prothese dar. Durch die nicht nur in einer Ebene, sondern mehrfach beweglichen Menisci, die durch entsprechende Polyethylen-Inlays definiert sind, werden auch unerwünschte exzentrische Belastungen derselben vermieden. Die Menisci stellen sich in jeder Beugestellung des Knies optimal zum zugeordneten femoralen Gelenkelement einerseits und tibialen Lagerelement andererseits ein, ohne daß die Gefahr eines seitlichen Ausweichens besteht. Besonders vorteilhaft ist die allseitige Kompatibilität der Implantate, welche bei späterer weiterer Abnützung des Gegenkompartimentes eine Ergänzung bis zum Vollgelenk erlaubt, ohne noch gut sitzende tibiale Implantat-Teile entfernen zu müssen.

Des weiteren wird vorzugsweise darauf geachtet, daß nicht nur die knietypischen sagittalen Hüllkurven, sondern sämtliche Kurvaturen möglich Kreissektoren sind. Dies ist produktionstechnisch von besonderer Bedeutung. Damit wird eine hohe Beweglichkeit zum einen, aber eine ebenso hohe Stabilität des Knies zum anderen erzielt.

Nachstehend wird ein Ausführungsbeispiel eines erfindungsgemäßen Systems anhand der beigefügten Zeichnung näher erläutert. Es zeigen:
- Fig. 1: eine Hemi-Kniegelenk-Endoprothese in Vorderansicht;
- Fig. 2: die Hemi-Prothese gemäß Fig. 1 in Seitenansicht;
- Fig. 3: die Hemi-Prothese entsprechend Fig. 2 in gegenüber Fig. 2 geänderter Relativlage zwischen femoralem Gelenkelement und zugeordnetem Meniscus;
- Fig. 4: die Hemi-Prothese entsprechend Fig. 3 in Hinteransicht;
- Fig. 5: das tibiale Lagerelement samt Meniscus entsprechend Fig. 1 in Draufsicht;
- Fig. 6: den Meniscus für die Ausführungsform gemäß Fig. 1 in Stirnansicht;
- Fig. 7: ein femorales Gelenkteil samt zugeordnetem Patella-Gleitlager in Seitenansicht, und das Patella-Gleitlager zusätzlich in Vorderansicht;
- Fig. 8: das Patella-Gleitlager gemäß Fig. 7 in Draufsicht;
- Fig. 9: ein Doppelkufen-Femurschlitten mit Patella-Gleitlager in gebeugter Zuordnung zu zwei separaten Tibiahalbimplantaten in Ansicht von distal;
- Fig. 10: ein Patella-Gleitlager in Draufansicht bei gestrecktem Knie;
- Fig. 11: das Patella-Gleitlager gemäß Fig. 10 mit zugeordnetem Patella-Sphäroid in Ansicht von distal und teilweise im Querschnitt bei gebeugtem Knie;
- Fig. 12: die Anordnung gemäß Fig. 11 in Seitenansicht und teilweise im Längsschnitt;
- Fig. 13: das implantierte Patella-Sphäroid gemäß den Fig. 11 und 12 in Unteransicht bei gestrecktem Knie;
- Fig. 14: einen Doppelkufen-Femurschlitten mit Patella-Sphäroid in Beugung und in Ansicht von distal;
- Fig. 15a: ein Patella-Sphäroid in Unteransicht bei gestrecktem Knie;
- Fig. 15b: ein PE-Inlay für ein Patella-Sphäroid gemäß Fig. 15a in Unteransicht;
- Fig. 16a: ein Patella-Sphäroid teilweise im Querschnitt und teilweise in Ansicht bei gebeugtem Knie;
- Fig. 16b: ein PE-Inlay für ein Patella-Sphäroid gemäß Fig. 16a in Ansicht entsprechend Fig. 16a;
- Fig. 17: ein sphärisches Probedrehgleitlager mit Sicherungsring für ein PE-Inlay im Querschnitt;
- Fig. 18a, 18b: einen Längsschnitt und eine Seitenansicht des Patella-Sphäroids bzw. PE-Inlays gemäß den Fig. 16a, 16b;
- Fig. 19: ein tibiales Probe-Lagerelement in Draufsicht;
- Fig. 20: das Probe-Lagerelement gemäß Fig. 19 mit Probe-Meniscus in Seitenansicht gemäß Pfeil II in Fig. 19;
- Fig. 21: das Probe-Lagerelement gemäß Fig. 19 mit Probe-Meniscus in Seitenansicht gemäß Pfeil III in Fig. 19;
- Fig. 22: das Probe-Lagerelement gemäß Fig. 19 mit unterschiedlich hohen Probe-Menisci in Stirnansicht;
- Fig. 23: ein endgültig zu implantierendes tibiales Lagerelement in Draufsicht;
- Fig. 24: das Lagerelement gemäß Fig. 23 mit Meniscus in Seitenansicht gemäß Pfeil VI in Fig. 23;
- Fig. 25: das Lagerelement gemäß Fig. 23 in Seitenansicht gemäß Pfeil VII in Fig. 23;
- Fig. 26: das tibiale Lagerelement gemäß Fig. 23 mit unterschiedlich hohen Menisci in Stirnansicht;
- Fig. 27: einen Femurschlitten in Ansicht von distal; und
- Fig. 28: eine Kniegelenk-Endoprothese mit Femur - Doppelkufenschlitten und zwei unabhängig voneinander plazierten tibialen Implantaten in schematischer Vorderansicht und Streckstellung.

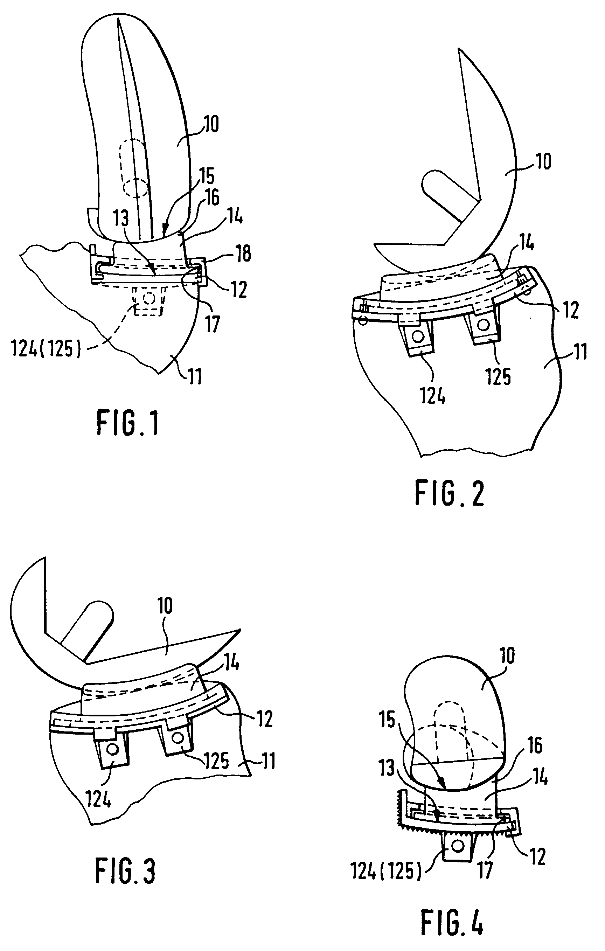

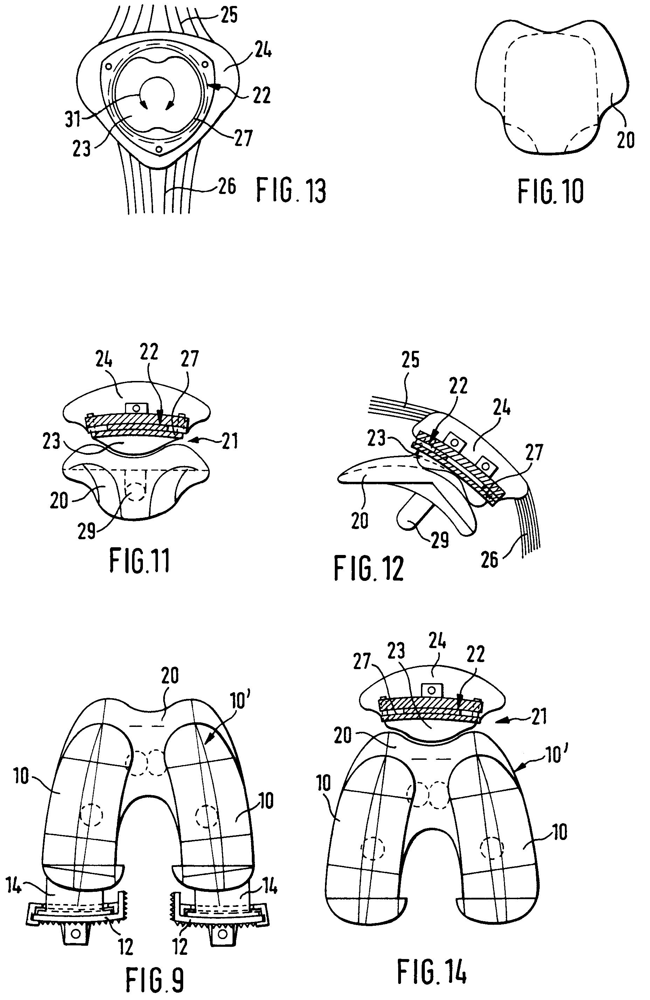

Die Figuren 1 bis 4 zeigen eine Hemi-Kniegelenk-Endoprothese in Beugestellung (Fig. 1 und 2) bzw. Streckstellung (Fig. 3 und 4). Dementsprechend ist am unteren Ende eines Oberschenkels bzw. Femur ein Gelenkelement 10 mit konvex gekrümmter Gelenklagerfläche angeordnet, während am oberen Ende des Schienbeins bzw. der Tibia 11 ein Lagerelement 12 mit sphärisch-konkav gewölbter Lagerfläche 13 befestigt bzw. verankert ist. Zwischen dem femoralen Gelenkelement 10 und dem tibialen Lagerelement 12 ist ein Meniscus 14 sowohl seitlich als auch antero-posterior beweglich angeordnet. Jeder Meniscus ist auf seiner Oberseite und seiner Unterseite mit Gleitflächen versehen, welche krümmungsmäßig komplementär zu der zugeordneten Gelenklagerfläche des femoralen Gelenkelements 10 bzw. der zugeordneten Lagerfläche 13 des tibialen Lagerelements 12 gestaltet sind. Die obere Gleitfläche des Meniscus 14 ist mit dem Bezugszeichen 15 gekennzeichnet. Der Meniscus 14 besteht aus Polyethylen. Das tibiale Lagerelement 12 ist am Knochen durch hülsenförmige Verankerungselemente 124, 125 mit jeweils einer Querbohrung verankert. Wie bereits in der Figurenaufzählung erwähnt, zeigen die Fig. 1 und 2 den femoralen Halbschlitten 10 in Beugelage, während sich in den Fig. 3 und 4 der Halbschlitten in Strecklage befindet. Die in den Fig. 1 bis 4 dargestelle femorale Halbprothese kann im nachhinein im Wege einer späteren Operation durch eine Vollprothese bzw. auch Revisionsprothese ersetzt werden. In diesem Fall sind der linke bzw. innere und rechte bzw. äußere femorale Kalbschlitten 10 einstückig miteinander verbunden. Diese Ausführungsform eines Femurschlittens ist in Fig. 9 in Zuordnung zu zwei separaten Tibiahalbimplantaten dargestellt und dort mit der Bezugsziffer 10' gekennzeichnet.

Wie die Fig. 1, 4 und 6 sehr gut erkennen lassen, ist die sagittal äußere Begrenzung der dem femoralen Gelenkelement 10 zugewandten Gleitfläche 15 des Meniscus 14 höher als deren sagittal innere Begrenzung. Die sagittal äußere Begrenzung ist in den Fig. 1, 4 und 6 jeweils mit der Bezugsziffer 16 gekennzeichnet. Durch diese Maßnahme ist das Kniegelenk seitlich nach außen hin abgestützt und insgesamt im Vergleich zum Stand der Technik stabiler.

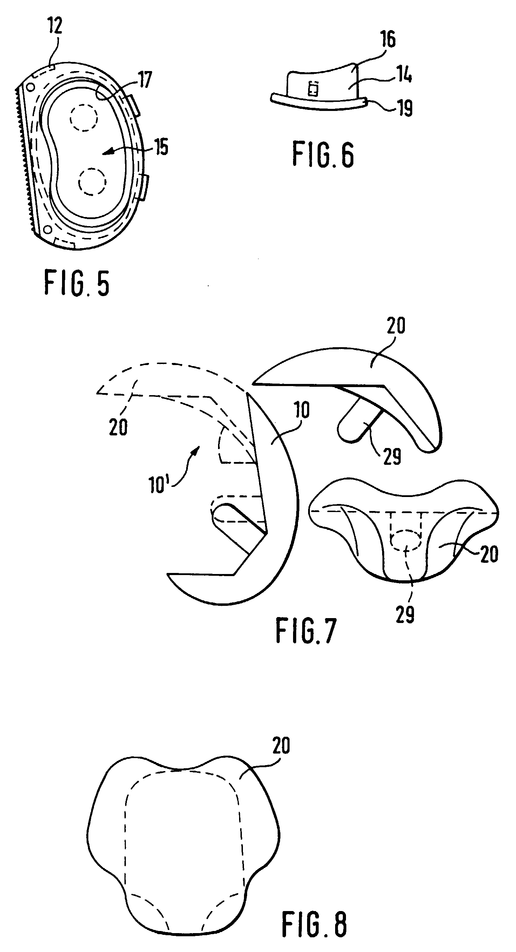

Wie bereits oben kurz dargelegt, sind sowohl die Lagerfläche der tibialen Lagerelemente 12 als auch die oberen Gleitflächen 15 der Menisci 14 jeweils sphärisch-konkav gewölbt. Damit wird eine hohe Beweglichkeit des Kniegelenks nach allen Richtungen hin erreicht, ohne daß die Stabilität desselben verlorengeht. Im Gegenteil, auch die Stabilität des Kniegelenks wird durch diese Maßnahmen zusätzlich erhöht. Vor allem erlaubt die vorgenannte Konstruktion eine Beweglichkeit der Menisci nicht nur antero-posterior, sondern auch zur Seite hin. Die Menisci stellen sich in jeder Relativlage des Kniegelenks optimal auf das femorale Gelenkelement 10 einerseite und das tibiale Lagerelement 12 andererseits ein. Um im Extremfall ein Herausrutschen oder Abheben eines Meniscus vom Lagerelement 12 zu vermeiden, weisen die tibialen Lagerelemente 12 an ihrer Oberseite eine nutartige Gleitführung 17 auf. Die nutartige Gleitführung 17 ist entsprechend Fig. 5 in Draufsicht um die Schienbein-Längsachse herum gebogen. Dadurch ist eine relative Verdrehung des Kniegelenks entsprechend der natürlichen Verdrehmöglichkeit gegeben. Entsprechend Fig. 5 erstreckt sich die Gleitführung 17 um jeden Meniscus 14 herum und hält diesen nach allen Seiten hin auf der Lagerfläche 13 des zugeordneten Lagerelements 12. Die nutartige Meniscus-Gleitführung 17 ist bei der dargestellten Ausführungsform nach oben und zur Seite hin durch auf das tibiale Lagerelement 12 aufsteckbare, insbesondere aufschnappbare Führungsleisten 18 begrenzt. Die Führungsleisten 18 sind bei dem dargestellten Ausführungsbeispiel Teil eines über den seitlichen Rand des zugeordneten tibialen Lagerelements 12 schnappbaren Rings. Es wird diesbezüglich insbesondere auf Fig. 5 verwiesen. Der nutartigen Meniscus-Gleitführung 17 entsprechend weist der Meniscus 14 an seiner Unterseite einen seitlich vorspringenden Rand 19 auf, der sich in Draufsicht auf den Meniscus um diesen herum erstreckt. Dieser Umfangsrand 19 erstreckt sich in die komplementäre nutartige Meniscus-Gleitführung 17 hinein. In Zusammenwirkung des Umfangsrandes 19 mit der nutartigen Gleitführung 17 wird der Meniscus 14 sicher auf dem Lagerelement 12 gehalten, wobei aufgrund eines entsprechenden seitlichen Spiels zwischen Umfangsrand 19 und nutartiger Gleitführung 17 nicht nur die übliche antero-posteriore Beweglichkeit, sondern auch eine seitliche Beweglichkeit des Meniscus gewährleistet ist.

Des weiteren lassen die Fig. 1 bis 4 erkennen, daß die Unterseite des tibialen Lagerelements 12 ebenfalls sphärischkonvex gewölbt ist, und zwar vorzugsweise entsprechend der Oberseite des Lagerelements. Durch diese Maßnahme ist eine Implantation mit minimaler Knochenentfernung möglich. Das tibiale Lagerelement wird mittels eines Impaktors kortikal aufliegend in vorbestimmter Neigung nach vorne oder hinten und/oder zur Seite hin eingeschlagen, wobei durch Spongiosa-Impaktion ein sphärisch gewölbtes Implantat-Bett entsteht, welches an die Unterseite des Lagerelements angepaßt ist. Dieses Implantat-Bett kann auch mittels eines gesonderten Impaktors vorbereitet werden.

Wie eingangs dargelegt, können bei dem dargestellten System tibiale Probe-Lagerelemente mit Probe-Menisci zur Anwendung kommen, wobei an der Unterseite der Probe-Lagerelemente jeweils mindestens ein, insbesondere zwei oder mehr kleine Knochenhaftdorne vorgesehen sind. Vorteilhafterweise sind an der Unterseite eines tibialen Probe-Lagerelements die Knochenhaftdorne in Reihen angeordnet, insbesondere jeweils nahe der beiden sagittalen Längsränder des Probe-Lagerelements.

Sowohl die Probe- als auch die endgültig implantierten Menisci 14 können unterschiedliche Höhen aufweisen. Im übrigen ist es für die Praxis sinnvoll, jeweils mindestens drei unterschiedliche Größen sowohl für die Probe-Lagerelemente als auch die endgültig implantierten Lagerelemente zur Verfügung zu stellen.

Die Gleitflächen der Lagerelemente sind vorzugsweise glattpoliert.

Mit Hilfe der nicht sehr tief in den Knochen eindringenden Knochenhaftdorne kann ein Probe-Lagerelement ohne Beschädigung des Knochens an diesem provisorisch Halt finden so lange, bis die richtige Positionbzw. Ebene des Lagerelements und zugeordneten Meniscus relativ zum zugeordneten femoralen Gelenkelement gefunden ist. Zu diesem Zweck ist es unter Umständen erforderlich, das Probe-Lagerelement mehrmals zu entfernen, um die Knochen-Auflagefläche nachzuarbeiten und das Probe-Lagerelement wieder einzusetzen, bis letztlich die richtige Längs- und Querneigung des Lagerelements und die richtige Höhe des Meniscus gefunden worden ist. Dann wird durch eine in der Meniscus-Gleitfläche ausgebildete Bohrung eines Probe-Meniscus sowie des Probe-Lagerelements hindurch eine tibiale Aufnahmebohrung ausgebildet, vorzugsweise mittels eines Zylinderhohlmeißels. Diese tibiale Bohrung dient zur Aufnahme eines an der Unterseite des endgültig implantierten Lagerelements angeordneten Verankerungselements 124 bzw. 125, wie sie in den Fig. 1 bis 4 angedeutet sind. Das Probe-Lagerelement sowie die dazugehörigen Probe-Menisci sind im wesentlichen genauso ausgebildet wie die endgültig implantierten Teile. Das Probe-Lagerelement unterscheidet sich vom endgültig implantierten Lagerelement im wesentlichen nur durch die kleineren Knochenhaftdorne an der vorzugsweise planen Unterseite im Vergleich zu den tiefer in den Knochen eindringenden Verankerungselementen an der vorzugsweise konvex sphärischen Unterseite beim endgültig implantierten Lagerelement.

In Fig. 7 ist ein femoraler Doppelkufenschlitten 10' in Seitenansicht dargestellt. Am rückseitigen proximalen Ende kann ein Patella-Gleitlager 20 angeschlossen sein, welches in Fig. 7 oben in Seitenansicht und in Fig. 7 rechts unten in Vorderansicht dargestellt ist. In Fig. 8 ist das Patella-Gleitlager 20 in Draufsicht gezeigt. Insofern enthält das beschriebene Kniegelenk-Endoprothesensystem eine kompatible Femuropatellar-Gelenkprothese.

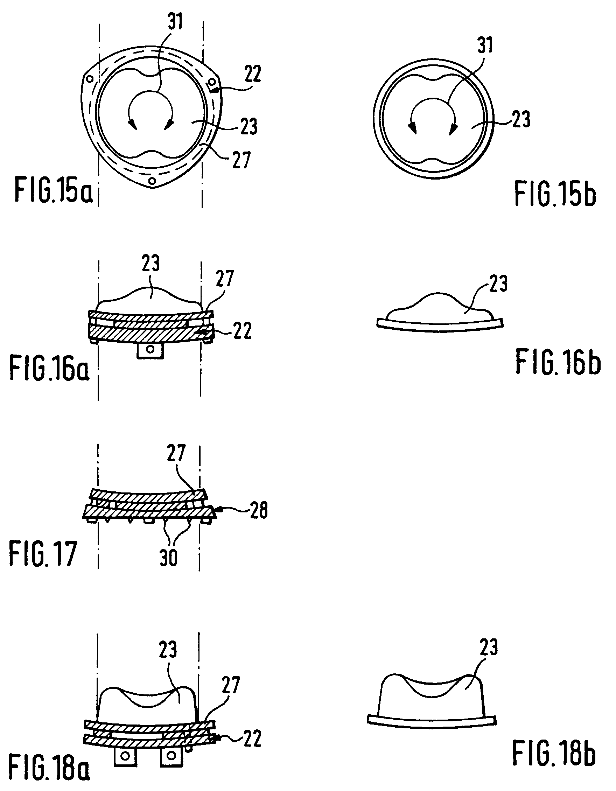

Anhand der Fig. 10 - 18b wird eine Ausführungsform einer Patella-Gleitlagerprothese als Teil des oben beschriebenen Systems näher erläutert. Dementsprechend besteht der femore-patellare Teil des System aus einem ein Patella-Gleitlager 20, das den Bereich zwischen den beiden femoralen Halbschlitten 10 (siehe auch die entsprechenden Ausführungen zu den Fig. 7 und 8) umfaßt und zu einem sogenannten Patella-Sphäroid 21, welches zwischen dem femoralen Patella-Gleitlager 20 und der knöchernen Patella (Kniescheibe) 24 plaziert ist, und zwar in fester Zuordnung an der Unterseite bzw. knieinneren Seite der Patella 24. Das Patella-Sphäroid 21 selbst besteht aus einem sphärischen bzw. Sphäroid-Drehgleitlager 22 für ein Stütz- und Gleitelement in Form eines Polyethylen- (PE) -Inlays 23, über das die Patella 24 sich am femoralen Patella-Gleitlager 20 abstützt. Es wird diesbezüglich insbesondere auf die Fig. 11 - 14 verwiesen. In den Fig. 12 und 13 sind auch noch die der Patella 24 zugeordneten Sehnen dargestellt, nämlich die Quadrizeps-Sehne 25 sowie Ligamentum patellae 26. Das PE-Inlay 23 wird auf dem sphärisch gewölbten Drehgleitlager 22 durch einen Schnappring 27 gehalten bzw. gesichert derart, daß die erforderliche Drehgleitbewegung des Inlays 23 relativ zum Drehgleitlager 22 nicht behindert ist. Die Drehgleitfläche des Drehgleitlagers 22 wird durch eine sphärisch gewölbte Metallplatte, insbesondere Platte z. B. aus Titanlegierung, definiert. An der der Drehgleitfläche abgewandten bzw. der Patella 24 zugewandten Seite sind Knochenverankerungselemente entsprechend den oben beschriebenen tibialen Verankerungselementen 124, 125 vorgesehen.

Die Verankerung des femoralen Patella-Gleitlagers 20 erfolgt durch einen femoralen Verankerungszapfen 29 (siehe Fig. 7 und 12). Die Drehgleitbewegung des PE-Inlays 23 ist in den Fig. 13, 15a, 15b durch den Rotations-Doppelpfeil 31 angedeutet.

In Fig. 17 ist noch ein sphärisches Probe-Drehgleitlager 28 mit Schnappring 27 dargestellt. Dieses Probelager unterscheidet sich von dem endgültig implantierten Drehgleitlager 22 durch relativ kleine Knochenhaftdorne 30, die gerade zur vorläufigen Sicherung des Probelagers 28 an der Patella 24 ausreichen. Des weiteren ist die der Patella zugewandte Anlagefläche flach, d. h. nicht konvex gewölbt ausgebildet, so wie dies bei dem endgültig implantierten Drehgleitlager 22 vorzugsweise der Fall ist. Die konvex gewölbte Knochenauflagefläche des Drehgleitlagers 22 entspricht nämlich der anatomischen Form der Restpatella 24, so daß keine großen Modifikationen der biomechanischen Hebelarme auftreten.

Die Kreis-Rotations-Freiheit des PE-Inlays 23 entsprechend dem Doppelpfeil 31 erlaubt eine kongruente Einpassung am femoralen Patella-Gleitlager 20 auch in den Stellungen, in denen die Zugrichtungen Patella-Quadrizeps und Patella-Tibia (Ligamentum patellae) sich nicht parallel zueinander und auch nicht parallel zur Furche des femoralen Patella-Gleitlagers 20 erstrecken.

Die Implantation des Patella-Sphäroids, insbesondere des sphärischen Drehgleitlagers 22 erfolgt vorzugsweise durch Einpressen an der Restpatella 24.

Das Probe-Drehgleitlager 28 dient in Analogie zu den unikompartimentalen Tibia-Implantaten zur seitlichen Zentrierung und zur Adaption der Implantations-Neigung in der Sagittalebene, ebenso wie entsprechende Bohrungen zur Aufnahme eines Zylinderhohlmeißels im Hinblick auf die Verankerungselemente am endgültig implantierten Patella-Lagerelement.

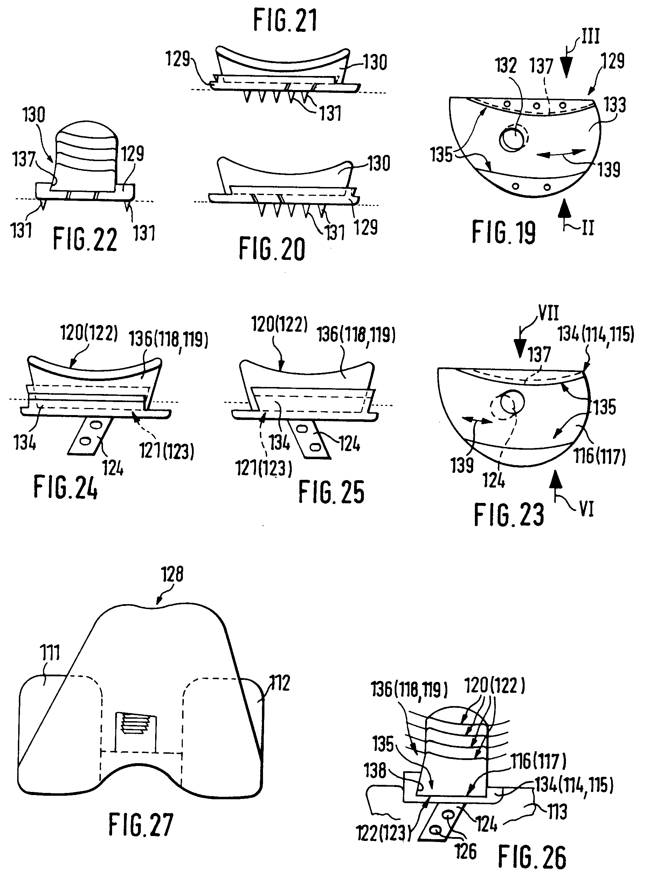

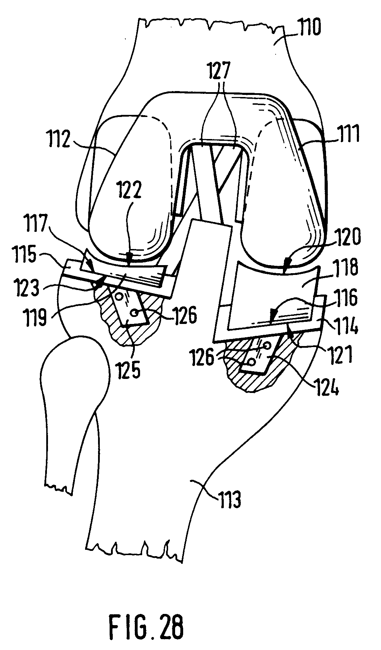

Anhand der Fig. 28 sollen nochmals die wesentlichen Teile einer Kniegelenk-Totalendoprothese mit dem beschriebenen System erläutert werden. Dementsprechend sind am unteren Ende eines Oberschenkels bzw. Femur 110 zwei Gelenkelemente (Gleithufen) 111, 112 mit jeweils konvex gekrümmter Gelenklagerfläche angeordnet, während am oberen Ende des Schienbeins bzw. Tibia 113 Lagerelemente 114, 115 mit ebener Lagerfläche 116, 117 befestigt bzw. verankert sind. Vorzugsweise sind die Lagerflächen 116, 117, wie oben ausgeführt, konkav sphärisch ausgebildet. Zwischen den femoralen Gelenkelementen 111, 112 und den tibialen Lagerelementen 114, 115 ist jeweils ein Meniscus 118, 119 beweglich angeordnet. Jeder Meniscus ist auf seiner Oberseite und seiner Unterseite mit Gleitflächen 120, 121 bzw. 122, 123 versehen, welche krümmungsmäßig komplementär zu der zugeordneten Gelenklagerfläche der femoralen Gelenkelemente 111 bzw. 112 bzw. der zugeordneten Lagerfläche des tibialen Lagerelements 114 bzw. 115 gestaltet sind. Die Menisci 118, 119 bestehen jeweils aus Polyethylen. Die tibialen Lagerelemente 114, 115 sind am Knochen durch hülsenförmige Verankerungselemente 124, 125 mit ein bzw. zwei Querbohrungen 126 verankert, wobei die Verankerungshülsen 124, 125 hier jeweils schräg zur Längsachse des Schienbeins 113 hin geneigt sind. Die Kreuzbänder sind in Fig. 28 mit der Bezugsziffer 127 gekennzeichnet. Im übrigen werden die hier interessierenden tibialen Implantate und das dazugehörige System zur Positionierung derselben anhand der Fig. 19 bis 26 näher erläutert. Zu Fig. 27 sei noch erwähnt, daß diese einen sogenannten Femurschlitten in Vorderansicht und in Strecklage zeigt, bei dem das innere und äußere Gelenkelement einstückig miteinander verbunden sind. Genauso gut ist jedoch eine getrennte Ausbildung denkbar, vor allem dann, wenn zunächst eine Halbprothese und im nachhinein im Wege einer späteren Operation eine Vollprothese bzw. auch Revisionsprothese implantiert wird. Diesbezüglich wird auch auf die Beschreibung der Fig. 1 - 9 verwiesen. Der Femur Vollschlitten ist in Fig. 27 mit der Bezugsziffer 128 gekennzeichnet.

In den Fig. 19 - 22 ist ein tibiales Probe-Lagerelement 129 mit Probe-Meniscus 130 dargestellt, wobei an der Unterseite des Probe-Lagerelements 129, die grundsätzlich flach ausgebildet ist, mehrere Knochenhaftdorne 131 vorgesehen sind. Die Knochenhaftdorne 131 sind entsprechend Fig. 22 jeweils längs der beiden Längsränder des Probe-Lagerelements in Reihe angeordnet. Mit Hilfe dieser nicht sehr tief in den Knochen eindringenden Dorne 131 kann das Probe-Lagerelement ohne Beschädigung des Knochens an diesem provisorisch verankert werden so lange, bis die richtige Position bzw. Ebene des Lagerelements und zugeordneten Meniscus relativ zum zugeordneten femoralen Gelenkelement gefunden ist. Zu diesem Zweck ist es unter Umständen erforderlich, das Probe-Lagerelement 129 mehrmals zu entfernen, um die Knochen-Auflagefläche nachzuarbeiten und das Probe-Lagerelement wieder einzusetzen, bis letztlich die richtige Längs- und Querneigung des Lagerelements und die richtige Höhe des Meniscus gefunden worden sind. Dann kann bzw. wird durch eine in der Meniscus-Gleitfläche 133 ausgebildete Bohrung 132 hindurch eine tibiale Aufnahmebohrung ausgebildet, vorzugsweise mittels eines Zylinderhohlmeißels. Diese tibiale Bohrung dient zur Aufnahme eines an der Unterseite des endgültig implantierten Lagerelements angeordneten stift-, laschen- oder hülsenförmigen Verankerungselements 124, welches in den Fig. 24, 25, 26 und 28 dargestellt ist. In den Fig. 23 - 26 ist mit der Bezugsziffer 134 das endgültig zu implantierende Lagerelement samt Meniscus 135 dargestellt, welches in Größe und Form dem Probe-Lagerelement samt Probe-Meniscus gemäß den Fig. 19 - 22 entspricht. Dementsprechend weisen sowohl die Probe-Lagerelemente 129 als auch die endgültig implantierten tibialen Lagerelemente 134 an ihrer Oberseite jeweils eine nutartige Gleitführung 135 für die Probe- sowie endgültig implantierten Menisci 130 bzw. 136 auf. Die nutartige Gleitführung 135 ist entsprechend den Fig. 19 und 23 in Draufsicht gebogen ausgebildet, und zwar um die Schienbein-Längsachse bzw. Kniequerachse herum. Dadurch ist eine relative Verdrehung des Kniegelenks entsprechend der natürlichen Verdrehmöglichkeit möglich.

Entsprechend den Fig. 22 und 26 ist wenigstens eine der beiden Längsseitenwände 137 bzw. 138 der nutartigen Gleitführung 135 in Richtung von oben nach unten schräg nach außen geneigt, wobei die zugeordnete Längsseitenwand der Probe-Menisci 130 bzw. der endgültig implantierten Menisci 136 eine komplementäre Neigung aufweist. Dadurch sind die Menisci vor einem unerwünschten Abheben vom Lagerelement gesichert. Die innere Längsseitenwand ist im übrigen etwas höher ausgebildet als die äußere Längsseitenwand, wie Fig. 26 sehr deutlich zeigt. Dadurch erhält man eine verbesserte Abstützung des Lagerelements am knöchernen Zentralpfeiler.

Entsprechend den Fig. 22 und 26 weisen die Probe-Menisci 130 ebenso wie die endgültig implantierten Menisci 136 unterschiedliche Höhen auf. Diese sind in den Fig. 22 und 26 mit den unterschiedlich hohen Gleitlagerflächen 120 angedeutet.

Wie bereits oben ausgeführt, weisen die den Probe-Lagerelementen 129 entsprechenden endgültig implantierten Lagerelemente 134 an ihrer Unterseite jeweils mindestens ein zapfen-, laschen- oder hülsenartiges Verankerungselement 124 (in Fig. 28 auch 125) mit mindestens einer Querbohrung 126 auf. Ein hülsenartiges Verankerungselement mit Querbohrung 126 hat den Vorteil, daß dieses optimal im Knochen einwächst. Außerdem muß bei der Implantation nur eine minimale Knochensubstanz entfernt werden, nämlich mittels des bereits erwähnten Zylinderhohlmeißels bzw. -fräsers. Entsprechend den Fig. 24 bis 26 und 28 erstrecken sich die Verankerungselemente 124 bzw. 125 schräg zur flachen Unterseite des Lagerelements 134, wobei der Winkel zwischen der flächennormalen und der Längsachse des Verankerungselements 124 bzw. 125 etwa 5 bis 20° beträgt. Im implantierten Zustand sind die Verankerungselemente vorzugsweise zur Längsachse der Tibia hin geneigt, so wie dies in Fig. 28 schematisch dargestellt ist.

Selbstverständlich sind den Probe-Menisci 130 entsprechende Menisci zur endgültigen Implantation zur Verfügung gestellt, so wie dies ein Vergleich der Fig. 22 mit Fig. 26 erkennen läßt. Im übrigen lassen Fig. 22 und 26 des weiteren erkennen, daS die Lagerelemente 129 bzw. 134 im Querschnitt jeweils wannenförmig ausgebildet sind. Damit läßt sich die Höhe der Menisci über die gesamte Breite derselben maximieren.

Für die Praxis ist es sinnvoll, jeweils drei unterschiedliche Größen sowohl für die Probe-Lagerelemente als auch die endgültig implantierten Lagerelemente zur Einlage der Menisci zur Verfügung zu stellen. Die Gleitflächen der Lagerelemente sind vorzugsweise glattpoliert.

Im übrigen ist es durch den wannenförmigen Querschnitt der Lagerelemente möglich, diese so tibial zu verankern, daß sie sowohl mit ihrer Unterseite als auch mit ihrer Innenseite am Knochen anliegen. Damit wird ein dauerhafter Halt der Lagerelemente im Knochen gewährleistet. Gegebenenfalls muß lediglich der Meniscus ausgetauscht werden, wobei der neue Meniscus hinsichtlich seiner Höhe an die zwischenzeitlich durch Beanspruchung und Abnutzung des Knies und/oder des anderen Meniscus geänderten Verhältnisse angepaßt werden kann. Der neue Meniscus soll wieder die ursprüngliche Seitenstabilität und korrekte Knie-Gesamtachse gewährleisten. In den Fig. 19 und 23 ist die Gleit-Beweglichkeit des Meniscus innerhalb der nutartigen Gleitführung 135 jeweils mit dem Doppelpfeil 139 gekennzeichnet.

## Patentansprüche

1. System für die Ausbildung einer Kniegelenk-Endoprothese, wobei das System versehen ist mit
- am unteren Ende des Oberschenkels (Femur) (110) befestigbaren femoralen Gelenkelementen (10, 10'; 111, 112) mit konvex gekrümmter Gelenklagerfläche,
- am oberen Ende des Schienbeins (Tibia) (11; 113) befestigbaren, separat ausgebildetenen, tibialen Lagerelementen (12; 114, 115, 134), und mit
- je einem zwischen den femoralen Gelenk- (10, 10'; 111, 112) und den tibialen Lagerelementen (12; 114, 115, 134) beweglich angeordneten Meniscus (14; 118, 119; 136) mit auf der Oberseite und der Unterseite ausgebildeten Gleitflächen (120, 121 bzw. 122, 123), welche komplementär zu der zugeordneten Gelenklagerfläche des femoralen Gelenkelementes (10, 10'; 111, 112) bzw. der zugeordneten Lagerfläche des tibialen Lagerelementes (12; 114, 115; 134) gestaltet sind,
**dadurch gekennzeichnet,**
daß die sagittal äußere Begrenzung (16) der den femoralen Gelenkelementen (10, 10'; 111, 112) zugewandten Gleitflächen (15; 120; 122) der Menisci (14; 118, 119; 136) bezüglich der Längsachse des Schienbeins (Tibia) (11; 113) höher liegt als deren sagittal innere Begrenzung und daß die Menisci (14; 118, 119; 136) unterschiedlich hoch ausgebildet sind.

2. System nach Anspruch 1,
dadurch gekennzeichnet,
daß das System vorläufig implantierte Probe-Menisci (130) aufweist, welche unterschiedlich hoch ausgebildet sind.

3. System nach einem der Ansprüche 1 bis 2,
dadurch gekennzeichnet,
daß die Lagerflächen (13) der tibialen Lagerelemente (12) jeweils sphärisch-konkav gewölbt sind.

4. System nach einem der Ansprüche 1 bis 3,
dadurch gekennzeichnet,
daß die tibialen Lagerelemente (12) an ihrer Oberseite mit einer nutartigen Gleitführung (17; 135) für die Probe- und ebenso die endgültig implantierten Menisci (14; 118, 119; 136) versehen sind.

5. System nach Anspruch 4,
dadurch gekennzeichnet,
daß die nutartige Gleitführung (17; 135) in Draufsicht um die Schienbein-Längsachse herum gebogen ist.

6. System nach einem der Ansprüche 1 bis 5,
dadurch gekennzeichnet,
daß die Unterseite der tibialen Lagerelemente (12) konvex, insbesondere entsprechend der Oberseite (13) derselben sphärisch-konvex gewölbt ist.

7. System, nach einem der Ansprüche 1 bis 6,
gekennzeichnet durch
tibiale Probe-Lagerelemente (129) mit Probe-Menisci (130), wobei an der Unterseite der Probe-Lagerelemente jeweils mindestens ein kleiner Knochenverankerungsdorn (131) vorgesehen ist.

8. System nach Anspruch 7,
dadurch gekennzeichnet,
daß an der (vorzugsweise planen) Unterseite der tibialen Probe-Lagerelemente jeweils nahe der beiden äußeren bzw. sagittalen Längsränder Knochenverankerungsdorne (131) angeordnet sind.

9. System nach einem der Ansprüche 5 bis 8,
dadurch gekennzeichnet,
daß mindestens eine der beiden Längsseitenwände (137 bzw. 138) der nutartigen Gleitführung (135) in Richtung von oben nach unten schräg nach außen geneigt ist, wobei die zugeordnete Längsseitenwand der Probe-Menisci (130) bzw. der endgültig implantierten Menisci (118, 119; 136) eine komplementäre Neigung aufweist.

10. System nach einem der Ansprüche 5 bis 9,
dadurch gekennzeichnet,
daß die nutartige Meniscus-Gleitführung (17) nach oben und zur Seite hin durch auf das tibiale Lagerelement (12) aufsteckbare, insbesondere aufschnappbare Führungsleisten (18) begrenzt ist, wobei bei sphärischkonkav gewölbter Lagerfläche (13) ein seitliches Spiel der Menisci (14) innerhalb der nutartigen Gleitführung (17) vorgesehen ist.

11. System nach Anspruch 10,
dadurch gekennzeichnet,
daß die Führungsleisten (18) der nutartigen Meniscus-Gleitführung (17) Teil eines über den seitlichen Rand des zugeordneten tibialen Lagerelements (12) schnappbaren Rings oder dergleichen Elementes sind.

12. System nach einem der Ansprüche 1 bis 11,
dadurch gekennzeichnet,
daß die femoralen Gelenkelemente Teil eines einteiligen Femurschlittens (10') sind, oder alternativ zwei voneinander getrennte Halbschlitten (10 bzw. 111, 112) bilden.

13. System nach einem der Ansprüche 1 bis 12,
dadurch gekennzeichnet,
daß neben den femoralen Gelenkelementen (10, 10'; 111, 112) ein Patella-Gleitlager (20) als Teil des Systems vorgesehen ist, insbesondere in vorbestimmter Zuordnung zu den femoralen Gelenkelementen (10, 10'; 111, 112).

14. System nach einem der Ansprüche 1 bis 13,
dadurch gekennzeichnet,
daß die den Probe-Lagerelementen (129) entsprechenden endgültig implantierten Lagerelemente (12; 114, 115; 134) an ihrer Unterseite jeweils mindestens ein hülsen-, zapfen- oder laschenförmiges Verankerungselement (124, 125), ggf. mit mindestens einer Querbohrung (126), aufweisen.

15. System nach Anspruch 14,
dadurch gekennzeichnet,
daß sich das Verankerungselement (124, 125) schräg zur flachen Unterseite des Lagerelements (114, 115; 134) erstreckt, wobei der Winkel zwischen der flächennormalen und der Längsachse des Verankerungselements (124, 125) etwa 5 bis 20° beträgt.

16. System nach Anspruch 15,
dadurch gekennzeichnet,
daß bei zwei oder mehr Verankerungselementen (124, 125) diese unterschiedlich stark gegenüber der flachen Unterseite des Lagerelements (114, 115; 134) geneigt sind.

17. System nach einem der Ansprüche 8 bis 16,
dadurch gekennzeichnet,
daß die Probe-lagerelemente (129) im Bereich ihrer Meniscus-Gleitfläche (133) jeweils eine Bohrung (132) zur Führung eines Knochenbearbeitungswerkzeuges, insbesondere eines Zylinderhohlmeißels, aufweisen.

18. System nach einem der Ansprüche 8 bis 17,
dadurch gekennzeichnet,
daß den Probe-Menisci entsprechende Menisci (14; 118, 119; 136) zur endgültigen Implantation bereitgestellt sind.

19. System nach einem der Ansprüche 14 bis 18,
dadurch gekennzeichnet,
daß dem Patella-Gleitlager (20) ein Patella-Sphäroid (21) mit einem zwischen der (knöchernen) Patella (24) und einem sich am femoralen Patella-Gleitlager (20) abstützenden Gleit- und Stützelement, insbesondere PE-Inlay (23) angeordneten sphärischen Drehgleitlager (22) zugeordnet ist, wobei dieses Drehgleitlager (22) mit hülsen-, zapfen- oder laschenförmigen Verankerungselementen zur Verankerung an der Patella (24) versehen ist.

20. System nach Anspruch 19,
gekennzeichnet durch
ein sphärisches Probe-Drehgleitlager (28), wobei an der der (knöchernen) Patella (24) zugeordneten Seite mindestens ein kleiner Knochenverankerungsdorn (30) vorgesehen ist, der gerade zur vorläufigen Sicherung des Probelagers (28) an der Patella (24) ausreicht.

## Claims

1. A system for producing a knee-joint endoprosthesis, whereby the system comprises
- femoral joint elements (10;10';111;112) adapted to be attached to the lower end of the femur (110) and each having an articular bearing surface with a convex curvature;
- separate tibial bearing elements (12;114;115;134) adapted to be attached to the upper end of the tibia (11;113); and with
- respective menisci (14;118;119;136) movably disposed between the femoral joint elements (10;10';111;112) and the tibial bearing elements (12;114;115;134) with sliding surfaces (15;120;121;or 122;133) formed at the upper side and the lower side, which are complementarily shaped to the associated joint bearing surface of the femoral joint element (10;10';111;112) respective the associated bearing surface of the tibial bearing element (12;114;115;134),
characterized in that
the sagittal outer boundary (16) of these sliding surfaces (15;120;122) of the menisci (14;118;119;136) that are facing the femoral joint elements (10;10';111;112) are located at a higher level than the level of their sagittal inner boundary relative to the longitudinal axis of the tibia (11;113) and that the menisci (14;118;119;136) are shaped differently high.

2. System according to claim 1, characterized in that the system is provided with trial menisci (130) to be implanted preliminarily which are shaped differently high.

3. System according to claim 1 or 2, characterized in that the bearing surfaces (13) of the tibial bearing elements (12) are each curved spherically-concave.

4. System according to one of the claims 1 to 3, characterized in that the tibial bearing elements (12) are provided with a groove-like sliding guide (17;135) at their upper side for receiving the trial menisci as well as the finally implanted menisci (14;118;119;136).

5. System according to claim 4, characterized in that the groove-like sliding guide (17;135) is configured to curve around the longitudinal axis of the tibia when viewed in the top view.

6. System according to one of the claims 1 to 5, characterized in that the lower side of the tibial bearing elements (12) is curved convex, particularly curved spherically-convex corresponding to its upper side (13).

7. System according to one of the claims 1 to 6, characterized in that tibial trial bearing elements with trial menisci are provided, whereby each of the trial bearing elements is provided with at least one small bone anchoring thorn (131) on the lower side.

8. System according to claim 7, characterized in that at the (preferably planar) lower side of the tibial trial bearing elements bone anchoring thorns (131) are configured in the vicinity of the two outer respectively sagittal longitudinal edges.

9. System according to one of the claims 5 to 8, characterized in that at least one of the two side walls (137 respectively 138) of the groove-like sliding guide (135) slants outward from top to bottom, whereby the mating longitudinal side wall of the trial menisci (130) respectively the finally implanted menisci (118;119;136) is slanted in a complementary manner.

10. System according to one of the claims 5 to 9, characterized in that the groove-like meniscus sliding guide (17) is limited upwardly and laterally by guiding bars (18) adapted to be attachable, particularly adapted for quick-snap action mounting onto the tibial bearing element, whereby in case of a bearing surface (13) with a spherical-concave curvature a lateral play of the menisci (14) within the groove-like sliding guide (17) is provided.

11. System according to claim 10, characterized in that the guiding bars (18) of the groove-like meniscus sliding guide (17) are part of a ring-like member or the like adapted for quick-snap action mounting over the lateral edge of the associated tibial bearing element (12).

12. System according to one of the claims 1 to 11, characterized in that the femoral joint elements are part of an integral femur sled (10') or alternatively two half-sleds (10 resp. 111;112) separated form one another.

13. System according to one of the claims 1 to 12, characterized in that apart from the femoral joint elements (10;10';111;112) a patella sliding bearing (20) is provided as part of the system, particularly in a predetermined allocation to the femoral joint elements (10;10';111;112).

14. System according to one of the claims 1 to 13, characterized in that the finally implanted bearing elements (12;114;115;134) corresponding to the trial bearing elements (129) are each provided at their lower sides with at least one anchoring element (124;125) shaped as a sleeve, pivot or clip if necessary with a transverse bore hole (126).

15. System according to claim 14, characterized in that the anchoring element (124;125) extends at an inclination relative to the flat lower surface of the bearing element (114;115;134), whereby the angle between the perpendicular to the surface and the longitudinal axis of the anchoring elements (124;125) has a value from substantially 5 to 20°.

16. System according to claim 15, characterized in that in case of two or more anchoring elements (124;125) these are slanted to different degrees with respect to the flat lower surface of the bearing element (114;115;134).

17. System according to one of the claims 8 to 16, characterized in that in the range of their meniscus sliding surface (133) the trial bearing elements (129) are each provided with a bore (132) for guiding a bone-working tool, particularly a cylindrical gouge.

18. System according to one of the claims 8 to 17, characterized in that menisci (14;118;119;136) for the final implantation and corresponding to the trial menisci are provided.

19. System according to one of the claims 14 to 18, characterized in that to the patella sliding bearing (20) includes a patella spheroid (21) with a spherical pivot sliding bearing (22) adapted to be disposed between the (bony) patella (24) and a sliding and support element, particularly PE-Inlay (23) supported on the femoral patella sliding bearing (20), whereby this pivot sliding bearing (22) is provided with anchoring elements shaped as sleeves, pivots or clips to be anchored at the patella (24).

20. System according to claim 19, characterized in that a spherical trial pivot slide bearing (28) is provided, whereby at least one small anchoring thorn (30) is provided on the side corresponding to the (bony) patella (24), the anchoring thorn (30) being just sufficient to temporarily secure the trial bearing (28) to the patella (24).

## Revendications

1. Système permettant de réaliser une endoprothèse pour l'articulation du genou, le système étant pourvu
- d'éléments articulés fémoraux (10, 10' ; 111, 112) ayant une surface d'appui articulé courbée de manière convexe qui peuvent être fixés à l'extrémité inférieure du fémur (110),
- des éléments d'appui tibial (12 ; 114, 115, 134), formés séparément, qui peuvent être fixés à l'extrémité supérieure du tibia (11 ; 113), et
- chaque fois d'un ménisque (14 ; 118, 119 ; 136), disposé de manière mobile entre les éléments articulés fémoraux (10, 10' ; 111, 112) et les éléments d'appui tibial (12 ; 114, 115, 134), ayant des surfaces de glissement (120, 121 ou encore 122, 123) formées sur la face supérieure et la face inférieure qui sont réalisées de manière complémentaire à la surface d'appui articulé coordonnée de l'élément articulé fémoral (10, 10' ; 111, 112) ou de la surface d'appui coordonnée de l'élément d'appui tibial (12 ; 114, 115, 134),
caractérisé en ce que
la limite sagittale extérieure (16) des surfaces de glissement (15 ; 120 ; 122) des ménisques (14 ; 118, 119 ; 136) faisant face aux éléments articulés fémoraux (10, 10' ; 111, 112) se trouve plus haut par rapport à l'axe longitudinal du tibia (11 ; 113) que leur limite intérieure et en ce que les ménisques (14 ; 118, 119 ; 136) ont des configurations de hauteur différente.

2. Système selon la revendication 1, caractérisé en ce que le système présente des ménisques d'essai (130) implantés temporairement qui ont des configurations de hauteur différente.

3. Système selon une des revendications 1 à 2, caractérisé en ce que les surfaces d'appui (13) des éléments d'appui tibial (12) sont respectivement arquées de manière sphériquement concave.

4. Système selon une des revendications 1 à 3, caractérisé en ce que les éléments d'appui tibial (12) sont pourvus sur leur face supérieure d'une coulisse (17 ; 135) en forme de rainure pour les ménisques d'essai et aussi pour les ménisques (14 ; 118, 119 ; 136) implantés de manière définitive.

5. Système selon la revendication 4, caractérisé en ce que la coulisse (17 ; 135) en forme de rainure vue du haut est incurvée autour de l'axe longitudinal du tibia.

6. Système selon une des revendications 1 à 5, caractérisé en ce que la face inférieure des éléments d'appui tibial (12) est courbé de manière convexe, en particulier d'une manière sphériquement convexe correspondant à la face supérieure (13) de ceux-ci.

7. Système selon une des revendications 1 à 6, caractérisé par des éléments d'appui tibial d'essai (129) avec des ménisques d'essai (130), une petite épine (131) d'ancrage osseux au moins étant chaque fois prévue sur la face inférieure des éléments d'appui d'essai.

8. Système selon la revendication 7, caractérisé en ce que des épines (131) d'ancrage osseux sont respectivement disposées sur la face inférieure (de préférence plane) des éléments d'appui tibial d'essai à proximité des deux bords longitudinaux extérieurs ou encore sagittaux.

9. Système selon une des revendications 5 à 8, caractérisé en ce qu'au moins une des deux parois latérales longitudinales (137 ou encore 138) de la coulisse (135) en forme de rainure est inclinée du haut vers le bas en oblique vers l'extérieur, la paroi latérale longitudinale coordonnée des ménisques d'essai (130) ou encore des ménisques implantés de manière définitive (118, 119 ; 136) présentant une inclinaison complémentaire.

10. Système selon une des revendications 5 à 9, caractérisé en ce que la coulisse (17) en forme de rainure pour le ménisque est limitée vers le haut et sur le côté par des barres conductrices (18) pouvant être emboîtées, en particulier encliquetées, sur l'élément d'appui tibial (12), un jeu latéral des ménisques (14) étant prévu à l'intérieur de la coulisse (17) en forme de rainure dans le cas d'une surface d'appui (13) arquée de manière sphériquement concave.

11. Système selon la revendication 10, caractérisé en ce que les barres conductrices (18) de la coulisse (17) en forme de rainure pour le ménisque font partie d'un anneau ou d'un élément semblable encliquetable le long du bord latéral de l'élément d'appui tibial (12) coordonné.

12. Système selon une des revendications 1 à 11, caractérisé en ce que les éléments articulés fémoraux font partie d'un traîneau fémoral (10') en une pièce, ou forment par ailleurs deux demi -traîneaux (10 ou encore 111, 112) séparés l'un de l'autre.

13. Système selon une des revendications 1 à 12, caractérisé en ce qu'à côté des éléments articulés fémoraux (10, 10' ; 111, 112), un palier de glissement (20) pour la rotule est prévu comme faisant partie du système, en particulier en coordination prédéterminée avec les éléments articulés fémoraux (10, 10'' 111, 112).

14. Système selon une des revendications 1 à 13, caractérisé en ce que les éléments d'appui implantés de manière définitive (12 ; 114, 115 ; 134) qui correspondent aux éléments d'appui d'essai (129) présentent respectivement sur leur face inférieure au moins un élément d'ancrage (124, 125) en forme de douille, de cheville ou de languette, le cas échéant avec au moins un orifice transversal (126).

15. Système selon la revendication 14, caractérisé en ce que l'élément d'ancrage (124, 125) s'étend en oblique par rapport à la face inférieure plate de l'élément d'appui (114, 115 ; 134), l'angle entre la normale du plan et l'axe longitudinal de l'élément d'ancrage (124,125) étant d'environ 5 à 20°.

16. Système selon la revendication 15, caractérisé en ce que dans le cas de deux éléments d'ancrage (124, 125) ou plus, ceux-ci sont inclinés à des degrés divers par rapport à la face inférieure plate de l'élément d'appui (114, 115 ; 134).

17. Système selon une des revendications 1 à 16, caractérisé en ce que les éléments d'appui d'essai (129) présentent respectivement dans la région de leur surface de glissement (133) pour le ménisque un orifice (132) pour guider un outil de façonnage des os, en particulier une gouge cylindrique.

18. Système selon une des revendications 1 à 17, caractérisé en ce que des ménisques (14 ; 118, 119 ; 136) correspondant aux ménisques d'essai sont préparés pour l'implantation définitive.

19. Système selon une des revendications 1 à 18, caractérisé en ce qu'un sphéroïde patellaire (21) comprenant un palier à glissement tournant (22) de forme sphérique disposé entre la rotule (24) (osseuse) et un élément de glissement ou de soutien s'appuyant sur le palier de glissement fémoral pour la rotule (20), en particulier un inlay en PE (23), est coordonné au palier de glissement de la rotule (20), ce palier à glissement tournant (22) étant pourvu d'éléments d'ancrage en forme de douille, de cheville ou de languette pour l'ancrage sur la rotule (24).

20. Système selon la revendication 19, caractérisé par un palier de glissement tournant d'essai (28) de forme sphérique, une petite épine d'ancrage osseux (30) au moins étant prévue sur la face coordonnée à la rotule (24) (osseuse), laquelle épine suffit juste pour la fixation temporaire du palier d'essai (28) à la rotule (24).
